# EUROPÄISCHE PATENTSCHRIFT

(11) **EP 2 219 566 B1**
(45) Veröffentlichungstag und Bekanntmachung des Hinweises auf die Patenterteilung: **14.08.2013**
(21) Anmeldenummer: 08839067.9
(22) Anmeldetag: 08.10.2008
(51) Int. Cl.: A61F 5/01

(54) **ORTHESENGELENK**
ORTHOSIS JOINT
ARTICULATION ORTHÉTIQUE

(30) Priorität: 17.10.2007 DE 202007014602 U
(43) Veröffentlichungstag der Anmeldung: 25.08.2010
(73) Patentinhaber: Gottinger Handelshaus oHG, 85604 Zorneding (DE)
(72) Erfinder: GÜNTHER, Norbert, G., 85599 Parsdorf (DE)
(74) Vertreter: Winter, Brandl, Fürniss, Hübner, Röss, Kaiser, Polte - Partnerschaft
(86) Internationale Anmeldenummer: PCT/DE2008/001659
(87) Internationale Veröffentlichungsnummer: WO 2009/049593

(56) Entgegenhaltungen:
- WO-A-2004/078078
- WO-A-2006/048012
- US-A1- 2005 187 505
- "Medizinisches Verordnungsprogramm" Februar 2002 (2002-02), GOTTINGER HANDELSHAUS , XP002512625 Seite 9 - Seite 10

## Beschreibung

Die Erfindung betrifft ein Orthesengelenk gemäß dem Oberbegriff des Anspruchs 1.

Ein derartiges Orthesengelenk wird beispielsweise unter dem Produktnamen "Sprinter" auf der Domain www.gottinger.de beschrieben. Es handelt sich dabei um ein Ein-Achs-Knöchelgelenk, das die Flexions- und Extensionsbewegung des Fußes in einem begrenzten Bereich zulässt. Dieses Knöchelgelenk findet seinen Einsatz beispielsweise in Unterschenkelorthesen und in Oberschenkel- oder beckenübergreifenden Gehorthesen. Um das Gelenk vorübergehend - beispielsweise postoperativ zu sperren oder wegen verbesserten Bewegungsverhältnissen die Fußstellung anzupassen, kann bei der bekannten Lösung ein auswechselbarer Bewegungsanschlag verändert werden. Dieser Bewegungsanschlag ist in eine Gelenkgabel des Knöchelgelenks eingesetzt, zwischen deren Gabelschenkel ein Gelenkbügel mittels eines Gelenkzapfens gelenkig gelagert ist. Der Bewegungsanschlag ist dabei so an der Gelenkgabel befestigt, dass der Gelenkbügel stirnseitig auf Anschlagflächen des Bewegungsanschlags aufläuft und somit den Bewegungsausschlag des Gelenks beidseitig, d. h. in Richtung der Plantarflexion und der Dorsalextension begrenzt, wobei der Bewegungsanschlag mit zwei entsprechenden Anschlagflächen ausgeführt ist, die entsprechend der freizugebenden Plantar- und Dorsalflexion zur Horizontalen angestellt sind.

Zum Auswechseln des Bewegungsanschlags muss der Gelenkbolzen zwischen der Gelenkgabel und dem Gelenkbügel gelöst werden, so dass dann der zwischen den beiden Gabelschenkeln angeordnete Bewegungsanschlag von der Gelenkgabel abgeschraubt und ausgewechselt werden kann. Diese Vorgehensweise ist äußerst umständlich, da die Orthese jeweils vom Patienten abgenommen und vergleichsweise umständlich demontiert werden muss. Dies ist auch dann erforderlich, wenn beispielsweise lediglich der die Plantar- oder Dorsalflexion begrenzende Anschlag verändert werden soll.

In der WO 2006/048012 A1 ist ein Orthesengelenk offenbart, bei dem der Bewegungsanschlag durch Auflaufen von Anschlagflächen des Gelenkbügels auf entsprechende Anschläge der Gelenkgabel gebildet ist. Bei einem Verschleiß muss dann entsprechend das Gabelstück oder der Gelenkbügel ausgewechselt oder nachbearbeitet werden.

Dem gegenüber liegt der Erfindung die Aufgabe zugrunde, ein Orthesengelenk zu schaffen, dessen Bewegungsausschlag auf einfache Weise veränderbar ist.

Diese Aufgabe wird durch ein Orthesengelenk mit den Merkmalen des Anspruchs 1 gelöst.

Erfindungsgemäß ist das Orthesengelenk mit einer Gelenkgabel ausgeführt, zwischen deren Gabelschenkel ein Gelenkbügel gelenkig gelagert ist. Der Bewegungsausschlag dieses Gelenks ist durch einen auswechselbaren Bewegungsanschlag begrenzt, der erfindungsgemäß zwei Anschlagkeile hat, die jeweils vom Außenumfang des Orthesengelenks her mit einem Anschlagträger der Gelenkgabel verbunden sind.

Durch die Zweiteilung des Bewegungsanschlags können diese individuell bei montiertem Gelenk ausgewechselt werden, so dass die Adaption äußerst schnell und einfach am Patienten selbst ohne Abnehmen der Orthese erfolgen kann.

Bei einem bevorzugten Ausführungsbeispiel der Erfindung bilden die Anschlagteile in einer Draufsicht (Richtung der Gelenkachse) etwa ein U, wobei im montierten Zustand ein Anschlagträger der Gelenkgabel - oder in kinematischer Umkehrung des Gelenkbügels -zwischen zwei durch Befestigungsabschnitte der Anschlagkeile ausgebildete U-Schenkel eintaucht. Die Befestigung der Anschlagkeile erfolgt dann auf einfache Weise durch Verschrauben dieser Befestigungsabschnitte mit dem mittigen Anschlagträger.

Das Auswechseln der mit hoher Präzision zwischen die Schenkel eingepassten Anschlagkeile erfolgt bei einem Ausführungsbeispiel durch eine Abziehschraube, die in den jeweiligen Anschlagkeil eingeschraubt wird und dann auf eine Stirnfläche des Anschlagträger aufläuft, so dass bei einem weiteren Einschrauben der Anschlagkeil etwa in Radialrichtung nach aussen aus der Gelenkgabel heraus bewegt wird.

Der anteriore- oder posteriore Anschlagkeil kann jeweils mit unterschiedlichen Anschlagwinkeln ausgeführt sein, um die Dorsal- und Plantarflexion entsprechend zu begrenzen.

Bei einem bevorzugten Ausführungsbeispiel wird das Orthesengelenk als Knöchelgelenk eingesetzt.

Die Anschlagkeile werden vorzugsweise aus einem weicheren Material als die Gelenkgabel und der Gelenkbügel ausgeführt, so dass deren Verschleiß minimiert ist. Als Material kann beispielsweise Messing oder dergleichen vorgesehen werden.

Sonstige vorteilhafte Weiterbildungen der Erfindung sind Gegenstand weiterer Unteransprüche.

Im Folgenden wird ein bevorzugtes Ausführungsbeispiel der Erfindung anhand schematischer Zeichnungen näher erläutert. Es zeigen:
Figur 1 eine Unterschenkelorthese mit einem erfindungsgemäßen Orthesengelenk;
Figur 2 das Orthesengelenk aus Figur 1 in einer Ansicht entlang einer Gelenkachse im teildemontierten Zustand;
Figur 3 eine Einzelteildarstellung des Knöchelgelenks aus Figur 2 und
Figur 4 eine Schnittdarstellung durch eine Gelenkgabel des Gelenks aus Figur 2.

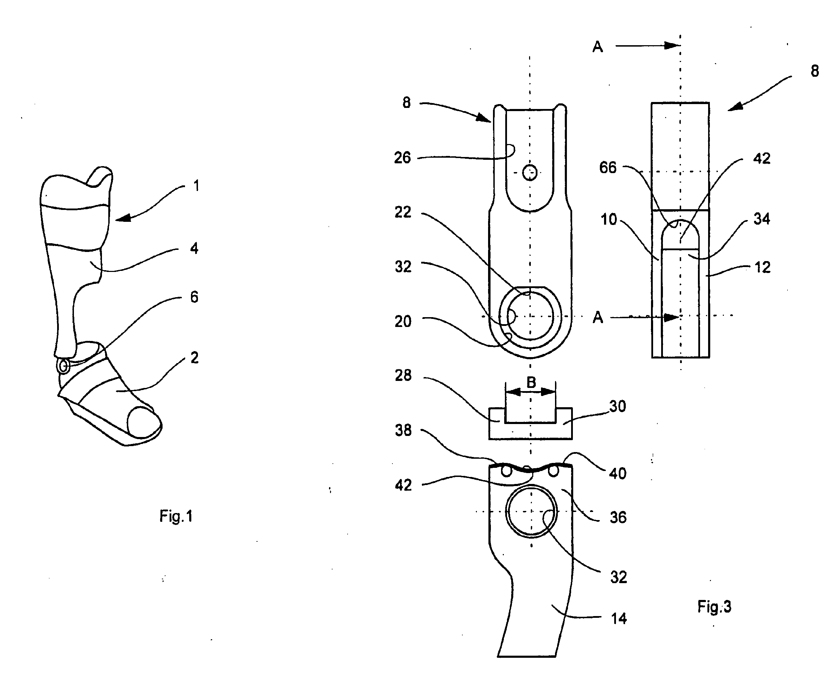

Figur 1 zeigt eine Unterschenkelorthese 1 mit einer Fußmanschette 2 und einer Unterschenkelmanschette 4, die mittels eines erfindungsgemäßen Knöchelgelenks 6 miteinander verbunden sind. Selbstverständlich ist die Erfindung nicht auf die Anwendung bei derartigen Knöchelgelenken begrenzt sondern kann prinzipiell bei allen Orhesengelenken eingesetzt werden.

Das Knöchelgelenk 6 lässt eine Flexion (nach unten) und Extensionsbewegung (nach oben) des von der Fußmanschette 2 ausgenommenen Fußes in einem begrenzten Bereich zu.

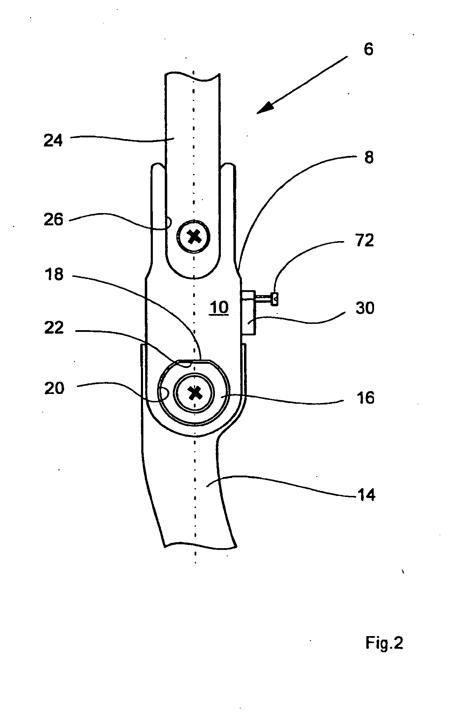

Figur 2 zeigt eine Einzeldarstellung des Knöchelgelenk 6 aus Figur 1. Demgemäß hat dieses eine Gelenkgabel 8, mit zwei Gabelschenkeln, von denen in der Darstellung gemäß Figur 2 nur der zum Betrachter weisende Gabelschenkel 10 sichtbar ist. Ein weiterer unterhalb der Zeichenebene liegender Gabelschenkel 12 ist in der folgenden noch näher beschriebenen Einzeldarstellung gemäß Figur 3 sichtbar.

Zwischen den beiden Gabelschenkeln 10, 12 der Gelenkgabel 8 ist ein Fußbügel 14 gelenkig gelagert. Dabei durchsetzt ein Gelenkbolzen 16 die beiden Gabelschenkel 10, 12 und den dazwischenliegenden Endabschnitt des Fußbügels 14, wobei der Gelenkbolzen 16 mittels einer Abflachung 18 drehfest in der Gelenkgabel 8 aufgenommen ist, deren den Gelenkbolzen 16 aufnehmende Aufnahme 20 mit einer entsprechenden Abflachung 22 zu sehen ist, so dass der Gelenkbolzen 16 drehfest in der Gelenkgabel 8 aufgenommen ist. Wie im folgenden noch näher erläutert, ist der Fußbügel 14 von einer Gelenkbohrung 32 durchsetzt und gleitend auf dem Gelenkbolzen 16 gelagert.

Der Fußbügel 14 ist über eine nicht dargestellte Fußstrebe mit der Fußmanschette 2 und die Gelenkgabel 8 über eine Unterschenkelmanschettenstrebe 24 mit der Unterschenkelmanschette 4 verbunden. Gemäß Figur 2 ist ein Gelenkabschnitt der Unterschenkelmanschettenstrebe 24 bündig in eine Aufnahmenut 26 der Gelenkgabel 8 eingesetzt.

Bis hierhin unterscheidet sich das erfindungsgemäße Knöchelgelenk nicht von der bekannten, eingangs beschriebenen Lösung.

Zur Begrenzung des Bewegungsausschlags des Fußbügels 14, sind zwischen den Gabeischenkeln 10, 12 der Gelenkgabel 8 zwei Anschlagkeile 28, 30 (siehe Figur 3) vorgesehen, von denen in der Ansicht gemäß Figur 2 nur der Anschlagkeil 30 sichtbar ist, da dieser aus seiner Wirkposition herausgezogen ist. Beim dargestellten Ausführungsbeispiel begrenzt der Anschlagwinkel 30 den Winkel der Dorsalflexion, während der andere, in Figur 3 sichtbare Anschlagkeil 28 die Plantarflexion begrenzt.

Die Einzelteile des Knöchelgelenks 6 werden nunmehr anhand Figur 3 erläutert. In Figur 3 oben sind eine Vorderansicht (in Richtung der Gelenkachse) und eine Seitenansicht der Gelenkgabel 8 dargestellt. Diese ist als Frästeil aus hochfestem Material, beispielsweise Titan hergestellt, wobei - wie bereits erläutert - die beiden Gabelschenkel 10, 12 von einer Gelenkbohrung 32 durchsetzt sind, die in beiden außenliegenden Großflächen der beiden Gabelschenkel 10, 12 zu den bereits anhand von Figur 2 beschriebenen Aufnahmen 20 für die Endabschnitte des Gelenkbolzens 16 erweitert ist. An dem von den Gabelschenkeln 10, 12 entfernten Endabschnitt der Gelenkgabel 8 sind beidseitig Aufnahmenuten 26 zur Befestigung der in Figur 2 dargestellten Unterschenkelmanschettenstrebe 24 ausgebildet. Der untere Endabschnitt der beiden Gabelschenkel 10, 12 ist - wie bei derartigen Gelenken üblich - abgerundet.

In einen von den beiden Gabelschenkeln 10, 12 abschnittsweise begrenzten Gabelraum 34 taucht ein Gelenkabschnitt 36 des Fußbügels 14 ein, der von der eingangs beschriebenen Gelenkbohrung 32 durchsetzt ist. Dabei ist der Gelenkabschnitt 36 mit hoher Präzision gefertigt, so dass er gleitend und spielfrei im Gabelraum 34 aufgenommen ist.

An der in Figur 3 obenliegenden, zur Gelenkgabel 8 weisenden Stirnfläche des Gelenkabschnitts 36 sind zwei etwa in Horizontalrichtung (in Figur 3) verlaufende Anschlagstirnflächen 38, 40 ausgebildet, die mit einem viskoelastischen Material als Dämpfer 42 beschichtet sind.

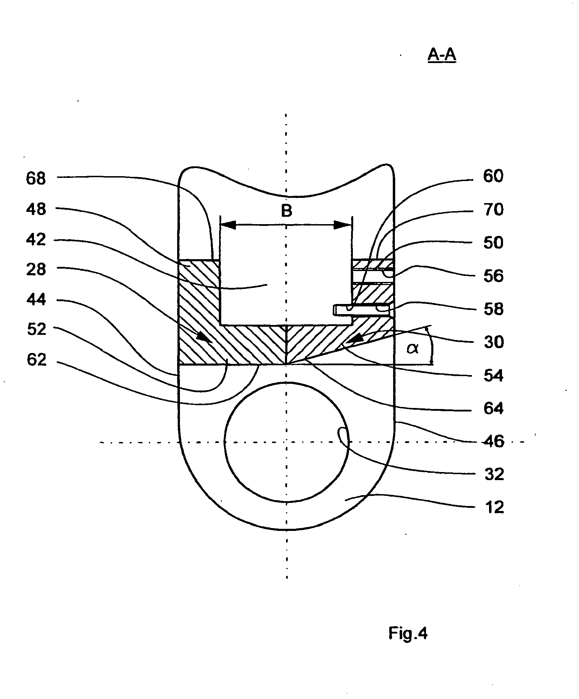

Wie anhand von Figur 4 noch näher erläutert wird, sind in den Boden des Gabelraums 34 die beiden gemeinsam ein U bildenden Anschlagkeile 28, 30 eingesetzt, die einen Bewegungsanschlag für den Fußbügel 14 bilden. Die Befestigung dieser beiden Anschlagkeile 28, 30 wird anhand Figur 4 erläutert, die einen Schnitt entlang der Linie A-A durch die Gelenkgabel 8 zeigen. Wie aus diesem Schnitt und der Seitenansicht gemäß Figur 3 entnehmbar ist, erstreckt sich entlang der Basis der beiden Gabelschenkel 10, 12 ein Anschlagträger 42 der jedoch gegenüber den in Figur 4 senkrecht zur Zeichenebene verlaufenden, seitlich verlaufenden Stirnflächen 44, 46 nach innen zurückversetzt ist. Die Breite B des Anschlagträgers 42 entspricht der lichten Weite B des durch die beiden Anschlagkeile 28, 30 ausgebildeten U.

Die beiden Anschlagkeile 28, 30 sind in der Ansicht gemäß Figur 4 jeweils L-förmig ausgebildet und haben jeweils einen Befestigungsabschnitt 48, 50 und eine Basis 52, 54, die gemeinsam jeweils eine Hälfte des Anschlagträgers 42 umgreifen und sich zu dem bereits erwähnten U ergänzen.

In jedem der Befestigungsabschnitte 48, 50 ist ein Abziehgewinde 56 und eine Befestigungsbohrung 58 ausgebildet, wobei letzterer jeweils ein Befestigungsgewinde 60 im Anschlagträger 42 zugeordnet ist. Das Ausziehgewinde wird vorzugsweise mit einem größeren Gewindedurchmesser als das Befestigungsgewinde 66 ausgeführt, so dass auch hohe Ausziehkräfte zum Herausziehen des Anschlagkeils 28, 30 ohne Beschädigung des Gewindes aufgebracht werden können.

Über die Befestigungsbohrung 58 und das zugeordnete Befestigungsgewinde 60 im Anschlagträger 42 können die beiden Anschlagkeile 28, 30 mit der Gelenkgabel 8 verschraubt werden.

An der in Figur 4 untenliegenden Stirnfläche der Basis 52, 54 der Anschlagkeile 28, 30 ist jeweils eine Anschlagfläche 62, 64 ausgebildet, deren Anstellwinkel α mit Bezug zur Horizontalen (Ansicht nach Figur 4) je nach dem gewünschten Bewegungsausschlag ausgebildet ist. Beim dargestellten Ausführungsbeispiel verläuft die Anschlagfläche 62 horizontal, so dass die zugeordnete Anschlagstirnfläche 38 des Fußbügels 14 flächig an dieser anliegt und entsprechend keine Plantarflexion zugelassen wird. Die Anschlagfläche 64 ist um den Winkel α angestellt, so dass eine entsprechende Dorsalflexion ermöglicht ist.

Soll nun aufgrund eines Verschleißes oder einer Veränderung des Bewegungsausschlages ein Anschlagkeil 28, 30 ausgewechselt werden, so wird die Befestigungsschraube gelöst und in die Abziehgewindebohrung 58 eine Abziehschraube 72 (siehe Figur 2) eingeschraubt, die dann auf die benachbarte, nicht mit einem Gewinde versehene Stirnfläche des Anschlagträgers 42 aufläuft. Beim weiteren Eindrehen der Abziehschraube 72 wird dann der entsprechende Anschlagkeil 28, 30 in der Figur 2 dargestellten Weise aus dem Gabelraum 34 herausgezogen und kann ohne Demontage des Knöchelgelenks 6 ausgewechselt oder angepasst werden. Dies ist gegenüber den herkömmlichen Lösungen ein erheblicher Fortschritt, da die Anpassung des Bewegungsausschlags am Patienten ohne Demontage der Orthese erfolgen kann.

Bei dem dargestellten Ausführungsbeispiel ist es bevorzugt, die beiden Anschlagkeile 28, 30 aus einem vergleichsweise weichem Material, beispielsweise aus Messing herzustellen.

Im montierten Zustand sitzen die beiden Anschlagteile 28, 30 bündig im Gabelraum 34, wobei sie nach außen hin mit den Stirnflächen 44, 46 abschließen und die Basis-Abschnitte 52, 54 mit den einander zuweisenden Stirnflächen aneinander anliegen. Des Weiteren liegen die Befestigungsabschnitte 48, 50 mit ihren inneren Stirnflächen flächig an den benachbarten Anlageflächen des Anschlagträgers 42 an. Bei dem dargestellten Ausführunsbeispiel ist die Basis zwischen den beiden Schenkeln 10, 12 mit einer Rundung 66 ausgeführt - die in der Darstellung gemäß Figur 4 oben liegenden Anlageflächen 68, 70 der Anschlagkeile 28, 30 sind dann entsprechend abgerundet, so dass auch in diesem Bereich eine flächige Anlage vorliegt, die Anschlagkeile 28, 30 nach dem Verschrauben kraft- und formschlüssig in der Gelenkgabel 8 aufgenommen sind.

Offenbart ist ein Orthesengelenk mit einer Gelenkgabel, zwischen deren Gabelschenkel ein Gelenkbügel gelenkig gelagert ist. Dessen Schwenkwinkel ist durch einen auswechselbaren Bewegungsanschlag begrenzt, der erfindungsgemäß durch zwei von aussen auswechselbare Anschlagkeile gebildet ist.

### Bezugszeichenliste:

- 1: Unterschenkelorthese
- 2: Fußmanschette
- 4: Unterschenkelmanschette
- 6: Knöchelgelenk
- 8: Gelenkgabel
- 10: Gabelschenkel
- 12: Gabelschenkel
- 14: Fußbügel
- 16: Gelenkbolzen
- 18: Abflachung
- 20: Aufnahme
- 22: Abflachung
- 24: Unterschenkelmanschettenstrebe
- 26: Aufnahmenut
- 28: Anschlagkeil
- 30: Anschlagkeil
- 32: Gelenkbohrung
- 34: Gabelraum
- 36: Gelenkabschnitt
- 38: Anschlagstirnfläche
- 40: Anschlagstirnfläche
- 42: Anschlagträger
- 44: Stirnfläche
- 46: Stirnfläche
- 48: Befestigungsabschnitt
- 50: Befestigungsabschnitt
- 52: Basis
- 54: Basis
- 56: Abziehgewindebohrung
- 58: Befestigungsbohrung
- 60: Befestigungsgewinde
- 62: Anschlagfläche
- 64: Anschlagfläche
- 66: Rundung
- 68: Anlagefläche
- 70: Anlagefläche
- 72: Abziehschraube

## Patentansprüche

1. Orthesengelenk mit einer Gelenkgabel (8), zwischen deren Gabelschenkeln (10, 12,) ein Gelenkbügel (14) gelenkig gelagert ist, dessen Bewegungsausschlag durch einen auswechselbaren Bewegungsanschlag begrenzt ist, **dadurch gekennzeichnet, dass** der Bewegungsanschlag zwei Anschlagkeile (28; 30) hat, die jeweils vom Aussenumfang her mit einem Anschlagträger (42) der Gelenkgabel (8) oder des Gelenkbügels (14) verbunden sind.

2. Orthesengelenk nach Anspruch 1, wobei die Anschlagkeile (28, 30) gemeinsam in einer Ansicht entlang der Gelenkachse ein U ausbilden, wobei der Anschlagträger (42) zwischen zwei U-Schenkel ausbildende Befestigungsabschnitte (48, 50) der Anschlagkeile (28, 30) eintaucht.

3. Orthesengelenk nach Anspruch 1 oder 2, wobei jeder Anschlagkeil (28, 30) mit dem Anschlagträger (42) verschraubt ist.

4. Orthesengelenk nach Anspruch 2 und 3, wobei im Abstand zur Verschraubung an jedem Befestigungsabschnitt (48, 50) eine Abziehbohrung (56) ausgebildet ist, in die eine Abziehschraube (72) einschraubbar ist, die mit ihrem Endabschnitt in Anlage an den Anschlagträger (42) bringbar ist.

5. Orthesengelenk nach einem der vorhergehenden Ansprüche, wobei stirnseitige Anschlagflächen (62, 64) der Anschlagkeile (28, 30) mit unterschiedlichen Anstellwinkeln (α) ausgebildet sind.

6. Orthesengelenk nach einem der vorhergehenden Ansprüche, wobei die Anschlagkeile (28, 30) aus Messing hergestellt sind.

7. Orthesengelenk nach einem der vorhergehenden Ansprüche, das als Knöchelgelenk ausgeführt ist.

## Claims

1. Orthosis joint having a joint fork (8), between the fork members (10, 12) of which there is supported in an articulated manner a curved joint member (14) whose movement amplitude is limited by a replaceable movement stop, **characterised in that** the movement stop has two stop wedges (28, 30) which are each connected from the outer periphery to a stop carrier (42) of the joint fork (8) or the curved joint member (14).

2. Orthosis joint according to claim 1, the stop wedges (28, 30) forming a U-shape together when viewed along the joint axis, the stop carrier (42) being introduced between two securing portions (48, 50) of the stop wedges (28, 30) that form two U-shaped members.

3. Orthosis joint according to claim 1 or 2, each stop wedge (28, 30) being screwed to the stop carrier (42).

4. Orthosis joint according to claim 2 and claim 3, there being formed with spacing from the screw connection at each securing portion (48, 50) a removal hole (56) in which there can be screwed a removal screw (72), which can be brought into abutment with the stop carrier (42) with the end portion thereof.

5. Orthosis joint according to any one of the preceding claims, end-face stop faces (62, 64) of the stop wedges (28, 30) being constructed so as to have different angles of incidence (α).

6. Orthosis joint according to any one of the preceding claims, the stop wedges (28, 30) being produced from brass.

7. Orthosis joint according to any one of the preceding claims, which is constructed as an ankle joint.

## Revendications

1. Articulation orthétique comprenant une fourche d'articulation (8), entre les branches d'articulation (10, 12) de laquelle est monté de manière articulée un étrier d'articulation (14), dont la déviation de mouvement est limitée par une butée interchangeable de déplacement, **caractérisée en ce que** la butée de déplacement comporte deux clavettes de butée (28, 30), lesquelles sont reliées respectivement depuis la périphérie extérieure à un support de butée (42) de la fourche d'articulation (8) ou de l'étrier d'articulation (14).

2. Articulation orthétique selon la revendication 1, sachant que les clavettes de butée (28, 30) forment conjointement selon une vue le long de l'axe d'articulation un U, sachant que le support de butée (42) s'enfonce entre deux sections de fixation (48, 50) des clavettes de butée (28, 30), lesquelles forment deux branches en U.

3. Articulation orthétique selon la revendication 1 ou 2, sachant que chaque clavette de butée (28, 30) est vissée au support de butée (42).

4. Articulation orthétique selon la revendication 2 et 3, sachant qu'est réalisé à distance par rapport au vissage au niveau de chaque section de fixation (48, 50) un alésage de pression (56), dans lequel une vis de pression (72) peut être vissée, laquelle peut être amenée par sa section d'extrémité en appui au niveau du support de butée (42).

5. Articulation orthétique selon l'une quelconque des revendications précédentes, sachant que des surfaces de butée (62, 64) côté frontal des clavettes de butée (28, 30) sont réalisées avec des angles d'incidence (α) différents.

6. Articulation orthétique selon l'une quelconque des revendications précédentes, sachant que les clavettes de butée (28, 30) sont fabriquées en laiton.

7. Articulation orthétique selon l'une quelconque des revendications précédentes, laquelle est réalisée sous la forme d'une articulation de cheville.
